# EUROPÄISCHE PATENTSCHRIFT

(11) **EP 2 495 462 B1**
(45) Veröffentlichungstag und Bekanntmachung des Hinweises auf die Patenterteilung: **22.08.2018**
(21) Anmeldenummer: 12157609.4
(22) Anmeldetag: 01.03.2012
(51) Int. Cl.: A61B 90/50, F16M 11/20, F16C 11/10

(54) **Einstellbare Haltevorrichtung für ein Endoskop**
Adjustable holding device for an endoscope
Dispositif de retenue réglable pour un endoscope

(30) Priorität: 01.03.2011 DE 102011004926
(43) Veröffentlichungstag der Anmeldung: 05.09.2012
(73) Patentinhaber: Karl Storz SE & Co. KG, 78532 Tuttlingen (DE)
(72) Erfinder: Abri, Omid, 14129 Berlin (DE); Reinauer, Josef, 72514 Inzigkofen (DE)

(56) Entgegenhaltungen:
- EP-A1- 1 152 182
- EP-A2- 1 959 184
- WO-A1-81/03054
- WO-A1-03/017859
- WO-A1-2008/040537
- DE-T2-602004 003 318
- US-A- 5 862 723
- US-A1- 2002 177 857
- US-A1- 2006 126 167
- US-B1- 6 632 170
- US-B1- 6 788 018

## Beschreibung

Die vorliegende Erfindung bezieht sich auf eine einstellbare Haltevorrichtung für ein Endoskop oder eine andere medizinische Einrichtung.

Bei Operationen und anderen medizinischen Eingriffen, bei denen ein Endoskop verwendet wird, kann es vorteilhaft sein, das Endoskop nicht manuell zu halten, sondern mittels einer Haltevorrichtung. Dadurch kann medizinisches Personal entlastet oder sogar eingespart werden. Ferner kann eine mechanische Haltevorrichtung ein Endoskop über einen beliebig langen Zeitraum präzise an einem bestimmten Ort und mit einer bestimmten Orientierung halten, ohne dabei zu ermüden.

In der DE 295 11 900 U1 ist eine Haltevorrichtung für chirurgische Zwecke beschrieben, die an einer Halteschiene eines Operationstisches festgelegt werden kann. Haltearme sind durch Kugelgelenke verschwenkbar miteinander verbunden. Jedem Kugelgelenk ist eine Feststellvorrichtung zugeordnet. Durch den Druck einer Schraubenfeder, die einen Topf gegen eine Kugel des Kugelgelenks drückt, wird die Kugel gegen Drehung in ihrem Lager festgelegt. Mittels eines Schließventils am vorderen Haltearm können die Feststellungen aller Kugelgelenke gleichzeitig gelöst werden. Mittels eines Lösehebels kann die Feststellung eines einzelnen Kugelgelenks gelöst werden.

In der WO 93/14704 ist eine Vorrichtung zum Positionieren eines chirurgischen Instruments beschrieben. Ein Kugelgelenk mit einer Kugel ist mittels eines pneumatischen Stellantriebs blockierbar. Zwischen dem pneumatischen Stellantrieb und einem auf die Kugel wirkenden Becher ist eine scherenartige Hebeleinrichtung zur Verstärkung der Kraft des pneumatischen Stellantriebs angeordnet.

Ein Nachteil einer Haltevorrichtung, wie sie beispielsweise in der DE 295 11 900 U1 beschrieben ist, besteht darin, dass beim abrupten und nicht dosierbaren Lösen der Feststellungen aller Kugelgelenke nur bei erheblicher Übung des medizinischen Personals eine ruckartige Bewegung der Haltevorrichtung aufgrund ihrer Schwerkraft und der Schwerkraft des gehaltenen Gegenstands vermieden werden kann.

In US 6,632,170 B1 ist ein gelenkiger Arm 10 zum Halten chirurgischer Instrumente beschrieben. Der Arm kann an einer Seitenschiene 14 an einem Operationstisch 16 befestigt werden und weist ein Gelenke 32 zum Schwenken des Arms 10 um eine vertikale Achse und zwei Gelenke 34, 36 zum Schwenken von Abschnitten 28, 30 des Arms um horizontale Achsen auf. Am distalen Ende des Arms 10 sind ein Kugelgelenk 44 und ein Druckknopf 50 vorgesehen. Die Gelenke 32, 34, 36, 44 sind jeweils durch Druckluft verriegelbar, wobei durch Druck auf den Druckknopf 50 der Druck manuell so weit abgesenkt werden kann, dass die Gelenke 32, 34, 36, 44 entriegelt sind. Zur Verriegelung des Kugelgelenks 13 ist ein Kolben 182 vorgesehen, der durch Druckluft und zusätzlich durch eine mechanische Feder 184 zur Kugel 176 des Kugelgelenks 44 gedrückt werden kann, um die Kugel 176 gegen einen spitzwinklig-konischen Sitz 180 zu drücken. An den übrigen Gelenken 30, 32, 34 ist zur Arretierung jeweils ein Kolben 130, der mittels Druckluft gegen die Kraft einer Feder 140 gegen einen spitzwinklig-konischen Sitz 138 gedrückt werden kann, vorgesehen. Am proximalen Gelenk 34 mit horizontaler Achse ist eine Vorspannungseinrichtung 188 mit einer Feder 192 zur teilweisen Kompensation des Gewichts des Arms 10 bei gelöster Arretierung vorgesehen.

In EP 1 152 182 Alist ein Operationsmikroskop, das an einem Stativ mit sechs Freiheitsgraden befestigt ist, beschrieben. Zur Höhenverstellung ist ein Parallelogrammarm 105 mit einer Gasfeder oder einem Federpaket 106 zum Gewichtsausgleich vorgesehen. Ferner sind mehrere vertikale Schwenkachsen A1, A2, A3 vorgesehen.

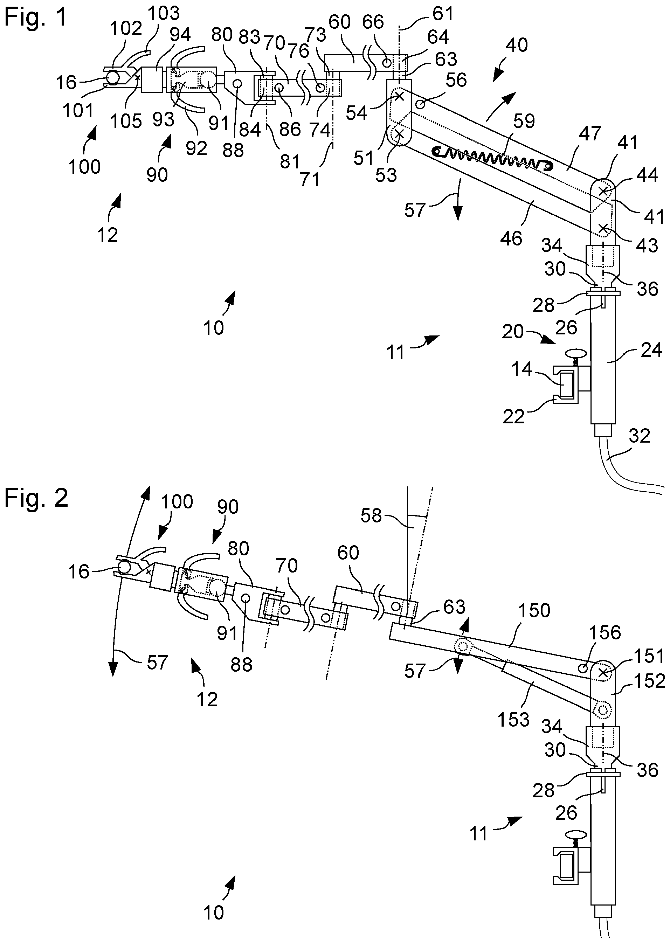

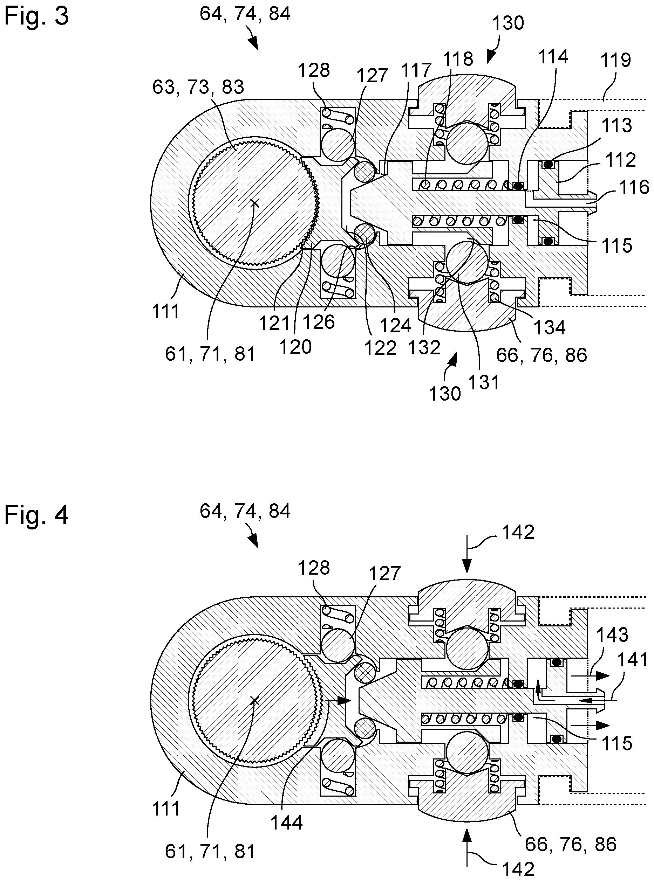

In WO 2008/040537 A1 ist ein chirurgisches Instrument mit einem mittels komprimierten Gases aus einer Gaskartusche verriegelbaren Kugelgelenk beschrieben (Seite 1, erster Absatz; Seite 2, Zeilen 4 bis 11; Fig. 1, 3a).

In US 5,862,723 ist ein Schraubenschlüssel mit mehreren Gelenken zwischen einem Kopfabschnitt und Griffabschnitten 70, 72 beschrieben. Die Gelenke ermöglichen ein zweifaches Abwinkeln des Schraubenschlüssels 10 und sind in bestimmten Winkeln arretierbar. Jedes Gelenk umfasst eine die Schwenkachse definierende Welle 47, einen manuell gegen die Kraft einer Feder 56 verschiebbaren Stift 22 mit einer gegenüber der Richtung seiner Verschiebbarkeit geneigten Fläche 24 und eine in einer Bohrung 52 verschiebbare Kugel 18. Die Feder 56 drückt den Stift 22 in eine Position, in der die geneigte Fläche 24 die Kugel 18 gegen eine gezahnte Oberfläche 36 drückt (Figur 5) und so formschlüssig das Gelenk arretiert.

Eine Aufgabe der vorliegenden Erfindung besteht darin, eine verbesserte Haltevorrichtung für ein Endoskop oder eine andere medizinische Einrichtung zu schaffen.

Diese Aufgabe wird durch den Gegenstand des unabhängigen Anspruchs 1 gelöst.

Weiterbildungen sind in den abhängigen Ansprüchen angegeben.

Eine einstellbare Haltevorrichtung für ein Endoskop oder eine andere medizinische Einrichtung umfasst ein proximales Ende, ein distales Ende mit einer Einrichtung zum Halten eines Endoskops oder einer anderen medizinischen Einrichtung, eine Mehrzahl von Armsegmenten zwischen dem proximalen Ende und dem distalen Ende, eine Mehrzahl von arretierbaren Gelenken, die jeweils zwei der Mehrzahl von Armsegmenten gelenkig miteinander verbinden, und eine Kompensationseinrichtung zum zumindest teilweisen Kompensieren der Gewichtskraft der Haltevorrichtung bei einem manuellen Schwenken oder Verschieben von zumindest einem Armsegment in vertikaler Richtung.

Das proximale Ende der einstellbaren Haltevorrichtung ist insbesondere zur Befestigung an einem Operationstisch ausgebildet. Dazu ist am proximalen Ende der Haltevorrichtung beispielsweise eine Klemmeinrichtung, die zu den an vielen Operationstischen seitlich vorgesehenen Stahlschienen kompatibel ist, vorgesehen.

Alternativ kann das proximale Ende der einstellbaren Haltevorrichtung zur mittelbaren oder unmittelbaren Befestigung an einer Decke eines Operationssaals oder eines anderen Raums ausgebildet sein. Insbesondere ist das proximale Ende der einstellbaren Haltevorrichtung ausgebildet, um an einer Deckenhalterung befestigt zu werden, die ihrerseits starr oder einstellbar ausgebildet sein kann.

Beispielsweise weist die einstellbare Haltevorrichtung an ihrem proximalen Ende einen Steckbolzen auf, der ausgebildet sein kann, um alternativ in die oben genannte Klemmeinrichtung zur Befestigung an einem Operationstisch oder in eine Deckenhalterung eingesetzt werden kann. Dazu weist der Steckbolzen insbesondere einen kreiszylindrischen oder im Wesentlichen kreiszylindrischen Abschnitt auf, der durch Klemmung oder auf andere Weise in einer entsprechenden Ausnehmung an der Klemmeinrichtung und/oder an der Deckenhalterung befestigt werden kann. Zur Unterstützung einer zuverlässigen Halterung - insbesondere für den Fall einer Befestigung an einer Deckenhalterung - kann der Steckbolzen in seinem kreiszylindrischen Abschnitt eine Kerbe, eine Nut (insbesondere eine umlaufende Nut) oder einen anderen konkaven Bereich aufweisen, der eine formschlüssige Verbindung ermöglicht.

Die Einrichtung zum Halten eines Endoskops oder einer anderen medizinischen Einrichtung am distalen Ende der einstellbaren Haltevorrichtung umfasst insbesondere zwei Backen, die durch eine Feder oder eine Schraube zusammengepresst werden können. Die beiden Backen sind insbesondere hinsichtlich Geometrie und Material an die typischen Querschnitte der Schäfte von Endoskopen angepasst, um diese sicher und ohne Beschädigung zu halten.

Ein Armsegment der Haltevorrichtung umfasst insbesondere einen geraden oder gekrümmten starren Abschnitt der Haltevorrichtung. Eine weitgehende Einstellbarkeit der Haltevorrichtung resultiert aus den arretierbaren Gelenken, die jeweils einen oder mehrere Freiheitsgrade aufweisen. Die Kompensationseinrichtung greift insbesondere an einem oder mehreren Armsegmenten und/oder an einem oder mehreren Gelenken an, um die Gewichtskraft des Armsegments an der Kompensationseinrichtung und/oder der Gewichtskraft von Armsegmenten distal der Kompensationseinrichtung zumindest teilweise zu kompensieren. Ferner kann die Kompensationseinrichtung ausgebildet sein, um die Gewichtskraft eines Endoskops oder einer anderen medizinischen Einrichtung zu kompensieren. Zur Verwendung mit unterschiedlichen Endoskopen oder anderen medizinischen Einrichtungen kann die Kompensationseinrichtung einstellbar sein, um den unterschiedlichen Massen und Gewichtskräften unterschiedlicher Endoskope und anderer medizinischer Einrichtungen Rechnung tragen zu können.

Mit einer Kompensation der Gewichtskraft ist insbesondere eine weitgehende Kompensation gemeint, so dass der nicht kompensierte oder überkompensierte Teil der Gewichtskraft nur einen geringen Anteil der Gewichtskraft ausmacht, beispielsweise höchstens 20 % oder höchstens 10 % oder höchstens 5 %. Die von der Kompensationseinrichtung ausgeübte Kompensationskraft beträgt somit zwischen 80 % und 120 % der Gewichtskraft bzw. zwischen 90 % und 110 % der Gewichtskraft bzw. zwischen 95 % und 105 % der Gewichtskraft. Insbesondere ist der Betrag der Differenz zwischen Gewichtskraft und Kompensationskraft so gering, dass er nicht zur Überwindung der Haftreibung in dem oder den entsprechenden Gelenken ausreicht, wenn dieses bzw. diese nicht arretiert sind.

Es ist offensichtlich, dass Gewichtskraft und Kompensationskräfte in den entsprechenden Gelenken entsprechende Drehmomente erzeugen, für die entsprechendes gilt.

Anders als eine einfache Arretierung eines Gelenks ist die Kompensationseinrichtung dazu vorgesehen und ausgebildet, auch bei einem Schwenken in vertikaler Richtung die Gewichtskraft zumindest teilweise zu kompensieren. Die Kompensationseinrichtung ist also eine von der Arretierung teilweise oder vollständig unabhängige Einrichtung, die auch bei gelöster Arretierung die Gewichtskraft der Haltevorrichtung bzw. von einem oder mehreren Armsegmenten und/oder eines Endoskops oder einer anderen medizinischen Einrichtung zumindest teilweise kompensieren kann.

Unten dargestellte Merkmale, Varianten und Ausführungsformen der einstellbaren Haltevorrichtung zeigen insbesondere, wie die einstellbare Haltevorrichtung so ausgestaltet werden kann, dass bereits eine einzige Kompensationseinrichtung an einem Armsegment oder an einem Gelenk eine zumindest teilweise Kompensation der Gewichtskraft der gesamten Haltevorrichtung ermöglicht. Alternativ können an mehreren Armsegmenten und/oder an mehreren Gelenken Kompensationseinrichtungen vorgesehen sein.

Die Kompensationseinrichtung der einstellbaren Haltevorrichtung ermöglicht es, dass beim Lösen der Arretierung der Gelenke nicht abrupt die gesamte Gewichtskraft der einstellbaren Haltevorrichtung, insbesondere ihrer distalen Armsegmente, und des Endoskops vom medizinischen Personal übernommen werden muss. Im Umgang mit einer herkömmlichen Haltevorrichtung antizipiert medizinisches Personal die Gewichtskraft und versucht diese im Moment des Lösens der Arretierung zu kompensieren. Dies setzt jedoch einerseits Geschicklichkeit und Übung des medizinischen Personals im Umgang mit der Haltevorrichtung und andererseits ein hohes Maß an Konzentration auf den Vorgang voraus. Jede Fehleinschätzung der Gewichtskraft bzw. jede Abweichung der tatsächlichen zu kompensierenden Gewichtskraft von der durch das medizinische Personal erwarteten hat eine abrupte Bewegung des distalen Endes der herkömmlichen Haltevorrichtung nach unten oder oben zur Folge. Wenn im gleichen Moment noch ein in eine natürliche oder künstliche Körperöffnung eines Patienten eingeführtes Endoskop durch die Haltevorrichtung gehalten wird, kann die resultierende abrupte Bewegung des Endoskops traumatisierend wirken.

Die Kompensationseinrichtung der vorliegenden verstellbaren Haltevorrichtung kann die nach dem Lösen der Arretierung vom medizinischen Personal zu kompensierende Gewichtskraft zumindest deutlich reduzieren. Bei geeigneter Dimensionierung oder Einstellung der Kompensationseinrichtung ist auch nach dem Lösen der Arretierung keine oder fast keine Kraft zum manuellen Halten des distalen Endes der einstellbaren Haltevorrichtung erforderlich. Unerwünschte Bewegungen des distalen Endes der einstellbaren Haltevorrichtung und insbesondere eines durch diese gehaltenen Endoskops können dadurch stark reduziert oder verhindert werden. Dies reduziert einerseits das Verletzungsrisiko für den Patienten und andererseits das Maß der seitens des medizinischen Personals erforderlichen Konzentration auf den Vorgang. Dadurch ermöglicht die Kompensationseinrichtung ein entspannteres und ermüdungsärmeres Arbeiten des medizinischen Personals und eine Verminderung oder vollständige Vermeidung einer Traumatisierung des Patienten durch ein unwillkürlich bewegtes Endoskop. Beides hat mittelbar und unmittelbar Vorteile für den Patienten und ökonomische Vorteile.

Viele herkömmliche Haltevorrichtungen beruhen mit zahlreichen Kugelgelenken auf dem Versuch einer möglichst weitgehenden Annäherung der Haltevorrichtung an den menschlichen Arm mit seinen zahlreichen Freiheitsgraden. Eine genauere Betrachtung zeigt allerdings, dass die als nahezu ideal angesehene hochgradige Beweglichkeit des menschlichen Arms dabei in der Regel gar nicht erreicht wird. Dies liegt vor allem daran, dass mit einem einzelnen Kugelgelenk ein Bewegungsbereich von deutlich mehr als ca. ± 40 Grad kaum erreichbar ist. Andererseits bietet eine herkömmliche Haltevorrichtung mit mehreren Kugelgelenken typischerweise zahlreiche Freiheitsgrade, die gar nicht benötigt werden. Beispielsweise weist ein Kugelgelenk in der Regel einen Freiheitsgrad der Rotation um die Symmetrieachse des Gelenks auf, der in der Regel allenfalls an einem oder zwei Gelenken der einstellbaren Haltevorrichtung erforderlich ist.

Insgesamt ermöglicht gerade ein zumindest weitgehender Verzicht auf die Verwendung von Kugelgelenken die einfachere Integration einer Kompensationseinrichtung, wie sie hier beschrieben ist. Einige nachfolgend beschriebene Merkmale und Ausführungsformen der Kompensationseinrichtung können auch an einem Kugelgelenk verwirklicht werden, allerdings nur mit deutlich höherem Aufwand.

Bei einer einstellbaren Haltevorrichtung, wie sie hier beschrieben ist, kann ein Armsegment in Gestalt einer Parallelogrammführung ausgebildet sein.

Insbesondere ist das äußerst proximale Armsegment oder das vom proximalen Ende der einstellbaren Haltevorrichtung aus erste nicht rein vertikal ausgerichtet Armsegment in Gestalt einer Parallelogrammführung ausgebildet. Die Parallelogrammführung ermöglicht eine vertikale Verschiebung des distalen Endes des in Gestalt einer Parallelogrammführung ausgebildeten Armsegments und der sich distal anschließenden Armsegmente.

Bereits mit einem einzigen in Gestalt einer Parallelogrammführung ausgebildeten Armsegment kann eine ausreichende vertikale Verschiebbarkeit des distalen Endes der einstellbaren Haltevorrichtung geschaffen werden, auch wenn weitere Armsegmente keine vertikalen Freiheitsgrade mehr aufweisen. Alternativ können zwei oder mehrere Armsegmente jeweils in Gestalt einer Parallelogrammführung ausgebildet sein.

Ein Vorteil der Parallelogrammführung besteht darin, dass das distale Ende des in Gestalt einer Parallelogrammführung ausgebildeten Armsegments gegenüber seinem proximalen Ende bei einer vertikalen Verschiebung lediglich vertikal und in gewissen Grenzen auch in horizontaler Richtung verschoben, jedoch nicht in vertikaler Richtung geschwenkt bzw. gekippt wird. Auch distal des in Gestalt einer Parallelogrammführung ausgebildeten Armsegments angeordnete Armsegmente werden deshalb nicht in vertikaler Richtung geschwenkt oder geneigt, sondern lediglich in vertikaler und horizontaler Richtung verschoben. Wenn die Kompensationseinrichtung an dem in Gestalt einer Parallelogrammführung ausgebildeten Armsegment vorgesehen ist, kann die von der Kompensationseinrichtung zu kompensierende Gewichtskraft deshalb von weiteren Freiheitsgraden der einstellbaren Haltevorrichtung unabhängig sein.

Insbesondere die Ausbildung von einem oder mehreren Armsegmenten in Gestalt einer Parallelogrammführung und die Anordnung von je einer Kompensationseinrichtung an jedem in Gestalt einer Parallelogrammführung ausgebildeten Armsegment ermöglicht somit eine besonders präzise Kompensation der Gewichtskraft und damit ein besonders feinfühliges manuelles Bewegen des distalen Endes der einstellbaren Haltevorrichtung.

Bei einer einstellbaren Haltevorrichtung, bei der ein Armsegment in Gestalt einer Parallelogrammführung ausgebildet ist, umfasst die Kompensationseinrichtung eine Feder oder ein anderes elastisches Element an dem in Gestalt einer Parallelogrammführung ausgebildeten Armsegment.

Insbesondere umfasst die Kompensationseinrichtung eine Feder oder ein anderes elastisches Element, das in dem in Gestalt einer Parallelogrammführung ausgebildeten Armsegment angeordnet ist, beispielsweise zwischen zwei parallelen Lenkern der Parallelogrammführung. Auch bei einer einstellbaren Haltevorrichtung, wie sie hier beschrieben ist, bei der kein Armsegment in Gestalt einer Parallelogrammführung ausgebildet ist, umfasst die Kompensationseinrichtung insbesondere eine Feder oder ein anderes elastisches Element. In allen Fällen kann die Feder eine Schrauben-, Spiral-, Blatt-, Gasdruck- oder andere Feder sein. Andere elastische Elemente sind beispielsweise elastisch verformbare Körper aus Gummi oder einem Elastomer. Die Anordnung einer Feder oder eines anderen elastischen Elements an einem in Gestalt einer Parallelogrammführung ausgebildeten Armsegment kann eine kostengünstig realisierbare und gleichzeitig präzise und robuste teilweise oder vollständige Kompensation der Gewichtskraft der Haltevorrichtung ermöglichen.

Bei einer einstellbaren Haltevorrichtung, wie sie hier beschrieben ist, umfasst die Kompensationseinrichtung insbesondere keinen motorischen Antrieb.

Insbesondere umfasst die Kompensationseinrichtung keinen elektrischen, magnetischen, hydraulischen oder pneumatischen motorischen Antrieb.

Bei einer einstellbaren Haltevorrichtung, wie sie hier beschrieben ist, sind insbesondere ein oder mehrere Armsegmente der Mehrzahl von Armsegmenten nur um vertikale oder im Wesentlichen vertikale Achsen schwenkbar.

Insbesondere bei Ausbildung eines Armsegments in Gestalt einer Parallelogrammführung, die eine vertikale Verschiebung ermöglicht, kann eine ausreichende Einstellbarkeit bzw. Bewegbarkeit der Haltevorrichtung, insbesondere des distalen Endes der Haltevorrichtung auch dann erreicht werden, wenn mehrere oder fast alle oder alle weiteren Armsegmente nur um vertikale oder im Wesentlichen vertikale Achsen schwenkbar sind. Die Ausbildung von einem oder mehreren Armsegmenten bzw. von zugeordneten Gelenken derart, dass das oder die Armsegmente lediglich um vertikale oder im Wesentlichen vertikale Achsen schwenkbar sind, erübrigt eine Kompensation der Gewichtskraft dieses oder dieser Armsegmente (sowie der distal davon angeordneten Armsegmente) an den entsprechenden Gelenken. Anders ausgedrückt ist eine Kompensation der Gewichtskraft lediglich an den Armsegmenten erforderlich, die nicht lediglich um vertikale oder im Wesentlichen vertikale Achsen schwenkbar sind. Im einfachsten Fall ist eine Kompensation lediglich an einem Armsegment erforderlich, das beispielsweise in Gestalt einer Parallelogrammführung ausgebildet ist. Dies ermöglicht gleichzeitig einen einfachen und kostengünstigen Aufbau der einstellbaren Haltevorrichtung und eine im Idealfall vollständige Kompensation der Gewichtskraft mit den oben dargestellten Vorteilen.

Bei einer einstellbaren Haltevorrichtung, wie sie hier beschrieben ist, sind insbesondere ein Gelenk an einem vorbestimmten Armsegment ausgebildet, um ein Schwenken um eine horizontale Achse zu ermöglichen, und weitere Gelenke jeweils lediglich für ein Schwenken um eine Achse, die senkrecht zu der horizontalen Achse ist, ausgebildet.

Im Fall der oben dargestellten Ausbildung eines Armsegments in Gestalt einer Parallelogrammführung sind die an dieses Armsegment angrenzenden Gelenke ausgebildet, um ein Schwenken um eine horizontale Achse zu ermöglichen. Wie bereits erwähnt, können weitere oder fast alle oder alle weiteren Gelenke jeweils lediglich für ein Schwenken um eine vertikale oder im Wesentlichen vertikale und damit zu der horizontalen Achse senkrechte Achse ausgebildet sein.

Alternativ ist ein vorbestimmtes Armsegment um eine horizontale Achse, insbesondere lediglich um eine horizontale Achse schwenkbar, und weitere Armsegmente sind mit dem vorbestimmten Armsegment bzw. untereinander durch Gelenke verbunden, die jeweils lediglich für ein Schwenken um eine Achse, die senkrecht zu der horizontalen Achse ist, ausgebildet sind. Je weiter distal ein Armsegment angeordnet ist, desto geringer ist die Auswirkung eines Schwenkens des Armsegments auf die Lage des Schwerpunkts der einstellbaren Haltevorrichtung. Je weiter proximal das vorbestimmte Armsegment angeordnet ist, das für ein Schwenken um eine horizontale Achse ausgebildet ist, desto geringer ist der Winkel, um den das vorbestimmte Armsegment und alle distal davon angeordneten Armsegmente um die horizontale Achse geschwenkt werden müssen, um eine vorbestimmte vertikale Verschiebung des distalen Endes der einstellbaren Haltevorrichtung zu erzielen.

Soweit Armsegmente distal des vorbestimmten Armsegments lediglich um geringe Winkel um ihre zugeordnete Achse geschwenkt werden müssen, verändert sich die Lage des Schwerpunkts der einstellbaren Haltevorrichtung auch nur geringfügig, und die Kompensationseinrichtung kann die Gewichtskraft der Haltevorrichtung weitgehend kompensieren. Soweit zur vertikalen Verschiebung des distalen Endes der einstellbaren Haltevorrichtung das vorbestimmte Armsegment und die Armsegmente distal des vorbestimmten Armsegments nur um einen kleinen Winkel um die horizontale Achse geschwenkt werden müssen, können die Abweichung der den Armsegmenten distal des vorbestimmten Armsegments zugeordneten Schwenkachsen von der vertikalen und die aus der Schwerkraft resultierenden Drehmomente gering sein. Bei einer einstellbaren Haltevorrichtung mit einem vorbestimmten Armsegment, das um eine horizontale Achse schwenkbar ist und weiteren Gelenken, die jeweils lediglich für eine Schwenken um eine Achse senkrecht zu der horizontalen Achse ausgebildet sind, kann somit die beim Lösen der Arretierung der Gelenke erforderliche Kraft zum Halten des distalen Endes der einstellbaren Haltevorrichtung und ggf. eines Endoskops daran gering sein.

Ein einstellbare Haltevorrichtung, wie sie hier beschrieben ist, kann anstelle eines in Gestalt einer Parallelogrammführung ausgebildeten Armsegments oder einer Schwenkbarkeit um eine proximal angeordnete horizontale Achse oder zusätzlich zu dieser eine vertikale Verschiebbarkeit mittels einer Linearführung, beispielsweise mittels eines teleskopierbaren vertikalen Armsegments, aufweisen. Auch in diesem Fall können weitere Armsegmente ausgebildet sein, um lediglich um vertikale oder im Wesentlichen vertikale Achsen schwenkbar zu sein. In diesem Fall ist die Kompensationseinrichtung an der vertikalen Linearführung angeordnet, so dass die Gewichtskraft der einstellbaren Haltevorrichtung in allen Positionen bzw. Konfigurationen der einstellbaren Haltevorrichtung teilweise oder vollständig kompensiert sein kann. Eine erfindungsgemäße Haltevorrichtung, wie sie hier beschrieben ist, umfasst ein arretierbares Gelenk eine Arretiereinrichtung zum Arretieren des Gelenks, eine Antriebseinrichtung zum Betätigen der Arretiereinrichtung und ein Getriebe, das eine kleinere Kraft und einen größeren Weg an der Antriebseinrichtung in eine größere Kraft und einen kleineren Weg an der Arretiereinrichtung übersetzt. Die Arretiereinrichtung umfasst beispielsweise eine glatte oder geriffelte Fläche, die an einer entsprechend ausgestalteten Oberfläche einer Welle anliegen kann, um eine relative Rotation der Welle und der Arretiereinrichtung zu unterbinden bzw. zu hemmen. Dazu ist die Arretiereinrichtung insbesondere in einer bezogen auf die Welle radialen Richtungen verschiebbar. Die Antriebseinrichtung umfasst beispielsweise einen Elektromotor, einen Elektromagneten, einen Hydraulikzylinder oder einen Pneumatikzylinder. Das Getriebe ist beispielsweise ein Zahnradgetriebe, ein Hebelgetriebe bzw. Koppelgetriebe oder ein Getriebe, bei dem an mehreren Flächen anliegende Gleitkörper Kräfte und Wege zwischen den Flächen übertragen können.

Die Verwendung eines Getriebes zwischen der Antriebseinrichtung und der Arretiereinrichtung ermöglicht beispielsweise im Fall einer pneumatischen oder hydraulischen Antriebseinrichtung die Verwendung eines geringeren Drucks zur Erzeugung einer vorbestimmten Kraft zum Betätigen der Arretiereinrichtung. Beispielsweise kann ein Getriebe die Verwendung der in Operationssälen üblicherweise bereitgestellten Druckluft mit einem vorbestimmten Druckniveau und/oder einem kleineren Querschnitt des Pneumatikzylinders ermöglichen.

Das Getriebe ist insbesondere ein Sperrgetriebe.

Die Verwendung eines Sperrgetriebes, bei dem das Über- bzw. Untersetzungsverhältnis und die Reibung innerhalb des Sperrgetriebes so dimensioniert sind, dass eine kleine Kraft am Eingang des Getriebes ausreicht, um auch bei einer großen Gegenkraft am Ausgang des Getriebes eine Bewegung zu unterbinden, kann insbesondere bei einer formschlüssig wirkenden Arretiereinrichtung vorteilhaft sein. Die Kraft, die zum Lösen bzw. Entriegeln der Arretierung erforderlich ist, kann in diesem Fall kleiner oder wesentlicher kleiner sein als die Kraft, die erforderlich ist, um die Arretierung zu überwinden.

Bei einer einstellbaren Haltevorrichtung mit einem Getriebe, wie sie hier beschrieben ist, kann das arretierbare Gelenk ferner eine Feder oder ein anderes elastisches Element umfassen, das so mit der Arretiereinrichtung gekoppelt ist, dass eine von dem elastischen Element ausgeübte Kraft eine Arretierung des Gelenks bewirkt.

In diesem Fall ist die Antriebseinrichtung zum Lösen der Arretierung ausgebildet. Bei Ausfall der für die Antriebseinrichtung erforderlichen Energie bzw. Leistung bleibt das arretierbare Gelenk arretiert. Beispielsweise bleibt die einstellbare Haltevorrichtung auch bei Ausfall der Druckluft arretiert. Eine erfindungsgemäße Haltevorrichtung, bei der ein arretierbares Gelenk eine Arretiereinrichtung, eine Antriebseinrichtung und ein Getriebe umfasst, umfasst das Getriebe insbesondere eine Gleitfläche an der Arretiereinrichtung, eine Gleitfläche an der Antriebseinrichtung, eine Führung und ein Gleitstück, das an der Gleitfläche der Arretiereinrichtung und an der Gleitfläche der Antriebseinrichtung anliegt und durch die Führung geführt ist.

Das Gleitstück ist beispielsweise ein kreiszylindrischer Körper. Die Führung ist beispielsweise eine weitere Gleitfläche an einem Gehäusekörper, der das Getriebe aufnimmt. Die Gleitflächen können jeweils eben oder gekrümmt sein. Jede Gleitfläche kann in der vorgesehenen Gleitrichtung des Gleitstücks gerade oder gekrümmt sein. Das Über- bzw. Untersetzungsverhältnis des Getriebes und ggf. seine Eigenschaften als Sperrgetriebe sind vor allem von den Winkeln zwischen den Gleitflächen und der durch die Führung vorgegebenen Richtung in den Bereichen, in denen das Gleitstück die Gleitfläche der Arretiereinrichtung, die Gleitfläche der Antriebseinrichtung und die Führung berührt, abhängig. Die Eigenschaften des Getriebes als Sperrgetriebe sind ferner von den tribologischen Eigenschaften der beteiligten Oberflächen abhängig, insbesondere von Haft- und Gleitreibung zwischen den beteiligten Oberflächen.

Wenn die Gleitfläche der Arretiereinrichtung, die Gleitfläche er Antriebseinrichtung jeweils eben und die Führung gerade sind, sind das Über- bzw. Untersetzungsverhältnis und ggf. die Eingenschaften des Getriebes als Sperrgetriebe von den Positionen der Arretiereinrichtung und der Antriebseinrichtung unabhängig. Durch eine Krümmung von einer oder beiden Gleitflächen an Arretiereinrichtung und Antriebseinrichtung und/oder durch eine Krümmung der Führung können eine Abhängigkeit des Über- bzw. Untersetzungsverhältnisses und ggf. eine Abhängigkeit der Eigenschaften als Sperrgetriebe von den Positionen der Arretiereinrichtung und der Antriebseinrichtung erreicht werden. Nachfolgend wird der Winkel zwischen der Flächennormalen der Gleitfläche der Arretiereinrichtung und der Flächennormalen der Gleitfläche der Antriebseinrichtung als erster Winkel bezeichnet. Der Winkel zwischen der Flächennormalen der Gleitfläche der Arretiereinrichtung und der Normalen auf die durch die Führung vorgegebene Richtung (insbesondere Flächennormale der die Führung bildenden Gleitfläche) wird als zweiter Winkel bezeichnet. Der Winkel zwischen der Normalen auf der durch die Führung vorgegebenen Richtung und der Flächennormalen der Gleitfläche an der Antriebseinrichtung wird als dritter Winkel bezeichnet. Mit der Flächennormalen ist jeweils die vom entsprechenden Körper weg zeigende Normale der Grenzfläche gemeint. Mit der Normalen auf der durch die Führung vorgegebenen Richtung ist jeweils die Normale gemeint, die mit der Flächennormalen der in Bezug genommenen Gleitfläche in einer Ebene liegt. Im Falle gekrümmter Gleitflächen und/oder einer gekrümmten Führung sind die Flächennormalen bzw. Normalen in den Bereichen gemeint, in denen das Gleitstück die Gleitflächen bzw. die Führung berührt.

Als vorteilhaft hat sich insbesondere eine Ausgestaltung des Getriebes derart erwiesen, dass der erste Winkel größer als der zweite Winkel ist und der zweite Winkel größer oder viel größer als der dritte Winkel ist. Ein Winkel ist insbesondere dann größer als ein anderer Winkel, wenn die Differenz der Winkel wesentlich ist, insbesondere mindestens 5 Grad oder mindestens 10 Grad oder mindestens 15 Grad beträgt. Ein Winkel ist insbesondere dann viel größer als ein anderer Winkel, wenn die Differenz der Winkel mindestens 30 Grad oder mindestens 40 Grad beträgt.

Der erste Winkel liegt insbesondere im Bereich von 130 Grad bis 170 Grad oder im Bereich von 140 Grad bis 160 Grad. Der zweite Winkel liegt insbesondere im Bereich von 110 Grad bis 150 Grad oder im Bereich von 120 Grad bis 140 Grad. Der dritte Winkel liegt insbesondere im Bereich von 60 Grad bis 100 Grad oder im Bereich von 70 Grad bis 90 Grad.

Das beschriebene Getriebe mit einem zwischen einer Führung und zwei Gleitflächen liegenden Gleitstück kann ein besonders einfach aufgebautes und robustes Sperrgetriebe zwischen der Antriebseinrichtung und der Arretiereinrichtung bilden. Dazu sind insbesondere die genannten Winkel und die Reibungswinkel für die jeweils aneinander gleitenden Materialpaarungen aufeinander abgestimmt. Insbesondere bei einer formschlüssigen Verriegelung durch Riffelungen an der Arretiereinrichtung und beispielsweise einer zugeordneten Oberfläche einer Welle kann das beschriebene Getriebe so ausgeführt werden, dass die von der Antriebseinrichtung zum Einrücken der Arretiereinrichtung aufgebrachte Kraft wesentlich kleiner ist als die Kraft, mit der die Arretiereinrichtung in der arretierenden Position gehalten wird.

Eine einstellbare Haltevorrichtung, wie sie hier beschrieben ist, kann eine Entriegelungseinrichtung mit einem Druckknopf, die einem einzigen arretierbaren Gelenk zugeordnet ist, zum Lösen der Arretierung des zugeordneten arretierbaren Gelenks durch manuellen Druck auf den Druckknopf, umfassen.

Insbesondere wenn die am Druckknopf aufgebrachte Kraft durch eines der oben beschriebenen Getriebe zwischen der Antriebseinrichtung und der Arretiereinrichtung verstärkt wird, kann eine verhältnismäßig kleine Kraft auf den Druckknopf ausreichen, um die Arretierung zu lösen. Dazu wirkt der Druckknopf insbesondere unmittelbar oder mittelbar auf die Antriebseinrichtung. Es kann vorteilhaft sein, zwei gegenüberliegende Druckknöpfe vorzusehen, die beide die Arretierung lösend auf die Antriebseinrichtung einwirken. Die beiden Druckknöpfe sind dann insbesondere so angeordnet, dass sie vom Daumen und vom Zeige- oder Mittelfinger einer Hand eines medizinischen Personals zusammengedrückt werden können, um die Arretierung des zugeordneten arretierbaren Gelenks zu lösen.

Das Lösen einer Arretierung eines Gelenks durch Druck auf einen Druckknopf oder gleichzeitigen Druck auf zwei Druckknöpfe kann besonders ergonomisch sein. Insbesondere ist damit beispielsweise im Vergleich zu der in der WO 93/14704 beschriebenen Hebeleinrichtung deutlich einfachere und schnellere Betätigung möglich.

Eine einstellbare Haltevorrichtung, wie sie hier beschrieben ist, kann eine Mehrzahl von Entriegelungseinrichtungen wie oben beschrieben umfassen, die je einem arretierbaren Gelenk zugeordnet sind.

Die einstellbare Haltevorrichtung ist ferner insbesondere ausgebildet, um mittels einer einzigen Steuereinrichtung alle oder fast alle arretierbaren Gelenke gleichzeitig zu arretieren bzw. alle oder fast alle Arretierungen gleichzeitig zu lösen. Mittels der Mehrzahl von Entriegelungseinrichtungen können zusätzlich die jeweils zugeordneten arretierbaren Gelenke einzeln manuell entriegelt werden. Die Mehrzahl von Entriegelungseinrichtungen kann somit eine Redundanz für den Fall des Ausfalls der Antriebseinrichtungen und eine zusätzliche Flexibilität bei der Arretierung bzw. beim Lösen der Arretierung einzelner Gelenke schaffen.

### Kurzbeschreibung der Figuren

Nachfolgend werden Ausführungsformen anhand der beigefügten Figuren näher erläutert. Es zeigen:
- Figur 1: eine schematische Darstellung einer einstellbaren Haltevorrichtung;
- Figur 2: eine schematische Darstellung einer weiteren einstellbaren Haltevorrichtung;
- Figur 3: eine schematische Darstellung eines arretierbaren Gelenks;
- Figur 4: eine weitere schematische Darstellung des arretierbaren Gelenks aus Figur 3.

### Beschreibung der Ausführungsformen

Figur 1 zeigt eine schematische Darstellung einer einstellbaren Haltevorrichtung 10 mit einem proximalen Ende 11 und einem distalen Ende 12. Die einstellbare Haltevorrichtung 10 ist ausgebildet, um am proximalen Ende 11 an einer Befestigungsschiene 14 an einem in Figur 1 nicht dargestellten Operationstisch befestigt zu werden und am distalen Ende 12 ein Endoskop 16 zu halten. Das Endoskop 16 wird in Figur 1 durch einen kreisförmigen Querschnitt eines Schafts beispielhaft dargestellt.

Am proximalen Ende 11 weist die einstellbare Haltevorrichtung 10 ein Haltesegment 20 mit einer Klemmeinrichtung 22 auf. Die Klemmeinrichtung 22 ist insbesondere als

Schraubklemme ausgebildet. Das Haltesegment 20 umfasst ferner ein vertikales Rohr 24, das mit der Klemmeinrichtung 22 verbunden, insbesondere verschweißt oder auf andere Weise starr verbunden, ist. Am oberen Ende weist das Rohr 24 einen oder mehrere parallel zu seiner Achse verlaufende Schlitze 26 und eine Spanneinrichtung 28 auf.

Die einstellbare Haltevorrichtung 10 umfasst ferner ein vertikales Armsegment 30, dessen unterer, rohrförmiger Abschnitt im vertikalen Rohr 24 des Haltesegments 20 angeordnet ist und dort in nahezu beliebiger Höhe mittels der Spanneinrichtung 28 arretiert werden kann. Das untere Ende des vertikalen Armsegments 30 ist mit einem Druckluftschlauch 32 verbunden. Zumindest der untere, im vertikalen Rohr 24 des Haltesegments 20 angeordnete Abschnitt des vertikalen Armsegments 30 kann als Druckreservoir für über den Druckluftschlauch 32 zugeführte Druckluft ausgebildet sein. Am oberen Ende des vertikalen Armsegments 30 ist ein Spannfutter 34 mit einer Symmetrieachse 36 vorgesehen.

Die einstellbare Haltevorrichtung 10 umfasst ferner ein als Parallelogrammführung ausgebildetes Armsegment 40. Das als Parallelogrammführung ausgebildete Armsegment 40 umfasst ein feststehendes Doppelgelenk 41, einen ersten Lenker 46, einen zweiten Lenker 47 und ein verschiebbares Doppelgelenk 51. Das gestrichelt angedeutete untere Ende des feststehenden Doppelgelenk 41 ist ausgebildet, um in dem Spannfutter 34 gehalten zu werden. Insbesondere ist das untere Ende des feststehenden Doppelgelenks 41 als Steckbolzen mit einem kreiszylindrischen oder im Wesentlichen kreiszylindrischen Abschnitt, der in dem Spannfutter durch Klemmung gehalten werden kann, ausgebildet. Wenn das Spannfutter 34 nicht arretiert ist, kann das feststehende Doppelgelenk 41 um die im Folgenden als vertikale Schwenkachse bezeichnete Symmetrieachse 36 des Spannfutters 34 geschwenkt werden.

Der erste Lenker 46 ist am feststehenden Doppelgelenk 41 um eine erste horizontale Schwenkachse schwenkbar gelagert. Der zweite Lenker 47 ist am feststehenden Doppelgelenk 41 um eine zweite horizontale Schwenkachse 44 schwenkbar gelagert. Am verschiebbaren Doppelgelenk 51 sind der erste Lenker 46 um eine dritte horizontale Schwenkachse 53 schwenkbar und der zweite Lenker 47 um eine vierte horizontale Schwenkachse 54 schwenkbar gelagert. Die erste horizontale Schwenkachse 43 und die zweite horizontale Achse 44 am feststehenden Doppelgelenk 41 und die dritte horizontale Schwenkachse 53 und die vierte horizontale Schwenkachse 54 am verschiebbaren Doppelgelenk 51 sind parallel zueinander. Der Abstand zwischen der ersten horizontalen Schwenkachse 43 und der zweiten horizontalen Schwenkachse 44 am feststehenden Doppelgelenk 41 und der Abstand zwischen der dritten horizontalen Schwenkachse 53 und der vierten horizontalen Schwenkachse 54 am verschiebbaren Doppelgelenk 51 sind gleich. Der durch den ersten Lenker 46 definierte Abstand der ersten horizontalen Schwenkachse 43 am feststehenden Doppelgelenk 41 und der dritten horizontalen Schwenkachse 53 am verschiebbaren Doppelgelenk 51 und der durch den zweiten Lenker 47 definierte Abstand der zweiten horizontalen Schwenkachse 44 am feststehenden Doppelgelenk 41 und der vierten horizontalen Schwenkachse am verschiebbaren Doppelgelenk 51 sind gleich. Dadurch bilden die horizontalen Schwenkachsen 43, 44, 53 ,54 in jeder Position der Lenker 46, 47 die Ecken eines Parallelogramms. Die Lenker 46, 47 sind um die horizontalen Schwenkachsen 43, 44 in der durch Pfeile 57 angedeuteten teilweise vertikalen Richtung schwenkbar. Dabei wird das verschiebbare Doppelgelenk 51 entsprechend verschoben.

Zwischen den Lenkern 46, 47 ist eine Zugfeder 59 in Form einer Schraubenfeder angeordnet. Die Lenker 46, 47 weisen insbesondere jeweils einen U-förmigen Querschnitt auf und sind so angeordnet, dass sie, wie in Figur 1 durch gestrichelte Linien angedeutet, überlappen. Die Zugfeder 59 ist insbesondere in dem durch die beiden U-förmigen Lenker 46, 47 umschlossenen Hohlraum angeordnet.

Der zweite horizontale Lenker 47 umfasst eine Arretierung zum unmittelbaren Arretieren der vierten horizontalen Schwenkachse 54 am verschiebbaren Doppelgelenk 51, und damit zum mittelbaren Arretieren des verschiebbaren Doppelgelenks 51 und zum mittelbaren Arretieren des feststehenden Doppelgelenks 41 und der ersten horizontalen Schwenkachse 43, der zweiten horizontalen Schwenkachse 44 und der dritten horizontalen Schwenkachse 53. Am zweiten horizontalen Lenker 47 sind zwei Druckknöpfe 56 zum Lösen der Arretierung der vierten horizontalen Schwenkachse 54 angeordnet, von denen in Figur 1 nur einer sichtbar ist.

Die einstellbare Haltevorrichtung 10 umfasst ferner ein erstes horizontales Armsegment 60, das um eine erste vertikale Schwenkachse 61 schwenkbar mit dem distalen Ende des als Parallelogrammführung ausgebildeten Armsegments 40 verbunden ist. Die erste vertikale Schwenkachse 61 des ersten horizontalen Armsegments 60 ist durch eine am verschiebbaren Doppelgelenk 51 angeordnete, insbesondere mit diesem starr verbundene, erste vertikale Welle 63 und eine erste Gelenkeinrichtung 64 am ersten horizontalen Armsegment 60 definiert. Die erste Gelenkeinrichtung 64 ist mit dem ersten horizontalen Armsegment 60 insbesondere starr verbunden oder integriert. Die zweite vertikale Welle 73 ist insbesondere mittels eines Gleit- oder Wälzlagers in der zweiten Gelenkeinrichtung 74 gelagert. Die erste Gelenkeinrichtung 64 ist mit der ersten vertikalen Welle 63 beispielsweise über ein Gleit- oder Wälzlager verbunden. Die erste vertikale Schwenkachse 61 ist insbesondere durch die Symmetrieachse der ersten vertikalen Welle 63 und des Lagers in der ersten Gelenkeinrichtung 64 definiert.

Die erste Gelenkeinrichtung 64 umfasst eine Arretierung zum Arretieren der ersten vertikalen Welle 63 in der ersten Gelenkeinrichtung 64. An der ersten Gelenkeinrichtung 64 sind zwei Druckknöpfe 66 zum Lösen einer Arretierung der ersten Gelenkeinrichtung 64 angeordnet, von denen in Figur 1 nur einer sichtbar ist.

Die einstellbare Haltevorrichtung 10 umfasst ferner ein zweites horizontales Armsegment 70, das um eine zweite vertikale Schwenkachse 71 schwenkbar mit dem ersten horizontalen Armsegment 60 verbunden ist. Die zweite vertikale Schwenkachse 71 des zweiten horizontalen Armsegments 70 ist durch eine mit dem distalen Ende des ersten horizontalen Armsegments 60 insbesondere starr verbundene zweite vertikale Welle 73 und eine zweite Gelenkeinrichtung 74 am zweiten horizontalen Armsegment 70 definiert. Die zweite Gelenkeinrichtung 74 ist mit dem proximalen Ende zweiten horizontalen Armsegment 70 insbesondere starr verbunden oder integriert. Die zweite vertikale Welle 73 ist insbesondere mittels eines Gleit- oder Wälzlagers in der zweiten Gelenkeinrichtung 74 gelagert. Die zweite vertikale Schwenkachse 71 ist insbesondere durch die Symmetrieachse der zweiten vertikalen Welle 73 und des Lagers in der zweiten Gelenkeinrichtung 74 definiert.

Die zweite Gelenkeinrichtung 74 umfasst eine Arretierung zum Arretieren der zweiten vertikalen Welle 73 in der zweiten Gelenkeinrichtung 74. An der zweiten Gelenkeinrichtung sind zwei Druckknöpfe 76 zum Lösen der Arretierung, von denen in Figur 1 nur einer sichtbar ist.

Die einstellbare Haltevorrichtung 10 umfasst ferner ein drittes horizontales Armsegment 80, das relativ zum distalen Ende des zweiten horizontalen Armsegments 70 um eine dritte vertikale Schwenkachse 81 schwenkbar ist. Die dritte vertikale Schwenkachse 81 ist durch eine mit dem dritten horizontalen Armsegment 80 insbesondere starr verbundene dritte vertikale Welle 83 und eine dritte Gelenkeinrichtung 84 am zweiten horizontalen Armsegment 70 definiert. bzw. durch die Symmetrieachse der dritten vertikalen Welle 83 und der dritten Gelenkeinrichtung 84 bestimmt. Die dritte Gelenkeinrichtung 84 ist mit dem distalen Ende des zweiten horizontalen Armsegments 70 insbesondere starr verbunden oder integriert. Die dritte vertikale Welle 83 ist insbesondere mittels eines Gleit- oder Wälzlagers in der dritten Gelenkeinrichtung 84 gelagert.

Die dritte Gelenkeinrichtung 84 ist ähnlich wie die erste Gelenkeinrichtung 64 am ersten horizontalen Armsegment 60 und die zweite Gelenkeinrichtung 74 am zweiten horizontalen Armsegment arretierbar und weist zwei Druckknöpfe 86 zum manuellen Lösen der Arretierung auf, von denen in Figur 1 nur einer sichtbar ist. Die dritte Gelenkeinrichtung 84 ist am distalen Ende des zweiten horizontalen Armsegments 70 angeordnet. Die dritte vertikale Welle 83 ist am dritten horizontalen Armsegment 80 angeordnet.

Am dritten horizontalen Armsegment 80, insbesondere nahe dem distalen Ende des dritten horizontalen Armsegments 80, ist ferner ein Steuerventil 88 angeordnet, das mittels eines oder zweier Druckknöpfe manuell betätigt werden kann.

Die einstellbare Haltevorrichtung 10 umfasst ferner ein weiteres, distales Armsegment 90, dessen proximales Ende über ein Kugelgelenk 91 mit dem distalen Ende des dritten horizontalen Armsegments 80 verbunden ist. Das distale Armsegment 90 umfasst zwei einander gegenüberliegende Entriegelungshebel 92, die mit einem im distalen Armsegment 90 angeordneten becherförmigen Klemmstück 93 mechanisch gekoppelt sind. Das becherförmige Klemmstück 93 wird durch eine in Figur 1 nicht dargestellte Feder oder ein anderes elastisches Element gegen die Oberfläche einer mit dem distalen Ende des dritten horizontalen Armsegments 80 verbundenen Kugel des Kugelgelenks 91 gedrückt, um das Kugelgelenk 91 zu arretieren. Durch Betätigen, insbesondere Zusammendrücken, der beiden Entriegelungshebel 92 kann das becherförmige Klemmstück 93 von der Oberfläche der Kugel des Kugelgelenks 91 abgehoben werden, um die Arretierung des Kugelgelenks 91 zu lösen.

Das distale Armsegment 90 weist an seinem distalen Ende eine Kupplung 94 auf. Das distale Armsegment 90 ist über die Kupplung 94 mit einer Klemmeinrichtung 100 lösbar verbunden. Anstelle der nachfolgend beschriebenen Klemmeinrichtung 100 kann eine andere Klemmeinrichtung, ein Adapter oder eine andere Einrichtung zum Halten eines Endoskops oder einer anderen medizinischen Einrichtung über die Kupplung 94 mit dem distalen Armsegment 90 verbunden werden.

Die Klemmeinrichtung 100 umfasst eine feststehende Greifbacke 101 und eine bewegbare Greifbacke 102. Die bewegbare Greifbacke 102 ist mit einem Griffhebel 103 starr verbunden oder mit diesem integral ausgebildet. Die bewegbare Greifbacke 102 und der Griffhebel 103 sind um eine Schwenkachse 105 schwenkbar. Eine in Figur 1 nicht dargestellte Feder oder ein anderes elastisches Element drückt die bewegbare Greifbacke 102 gegen die feststehende Greifbacke 101. Durch Betätigen, insbesondere Drücken, des Griffhebels 103 kann die bewegbare Greifbacke 102 gegen die Federkraft von der feststehenden Greifbacke 101 wegbewegt werden.

Bei der Darstellung in Figur 1 ist beispielhaft der kreisförmige Querschnitt eines Schafts eines Endoskops 16 zwischen der feststehenden Greifbacke 101 und der bewegbaren Greifbacke 102 geklemmt dargestellt. Um das Endoskop in unterschiedlichen Orientierungen zwischen der feststehenden Greifbacke 101 und der bewegbaren Greifbacke 102 klemmend halten zu können, kann an der feststehenden Greifbacke 101 und/oder an der bewegbaren Greifbacke 102 jeweils ein näherungsweise an den Querschnitt des Endoskops 10 angepasstes Profilelement um eine zur Längsachse des Endoskops 10 senkrechte und in der Zeichenebene der Figur 1 liegende Achse drehbar gelagert sein.

Die einstellbare Haltevorrichtung 10 weist eine Reihe von Freiheitsgraden auf. Ein Teil dieser Freiheitsgrade ist zur Grobjustage vorgesehen. Weitere Freiheitsgrade sind zur Feinjustage der einstellbaren Haltevorrichtung 10 vorgesehen.

Zur Grobjustage sind insbesondere die Klemmeinrichtung 22 am Haltesegment 20, die Spanneinrichtung 28 am vertikalen Rohr 24 und das Spannfutter 34 am vertikalen Armsegment 30 vorgesehen. Mittels der Klemmeinrichtung 22 kann das Haltesegment 20 an einem nahezu beliebigen Punkt an einer Befestigungsschiene 14 an einem Operationstisch befestigt und arretiert werden. Das vertikale Armsegment 30 kann im vertikalen Rohr 24 des Haltesegments 20 vertikal verschoben und in einer einstellbaren Höhe mit der Spanneinrichtung 28 arretiert werden. Die Spanneinrichtung 28 ist beispielsweise ähnlich einem Schnellspanner an einer Sattelstütze eines Fahrrads ausgebildet. Das untere Ende des feststehenden Doppelgelenks 41 des als Parallelogrammführung ausgebildeten Armsegments 40 kann in das Spannfutter 34 eingesetzt und um die vertikale Schwenkachse 36 rotiert werden. Mittels des Spannfutters 34 kann das als Parallelogrammführung ausgebildete Armsegment 40 hinsichtlich einer Rotation um die vertikale Schwenkachse 36 arretiert werden.

Die weiteren Freiheitsgrade der einstellbaren Haltevorrichtung 10 sind überwiegend für eine Feinjustage ausgebildet. Dazu können die Gelenkeinrichtungen überwiegend gemeinsam mittels eines zentralen Steuerelements wie dem bereits erwähnten Steuerventil 88 am dritten horizontalen Armsegment 80 oder jeweils einzeln arretiert oder die Arretierungen wieder gelöst werden. Zum Lösen der Arretierung eines einzelnen Gelenks 51, 64, 74, 84 sind insbesondere die Druckknöpfe 56, 66, 76, 86 und die Entriegelungshebel 92 vorgesehen. Die Arretierung und das Lösen der Arretierung mittels des Steuerventils 88 oder durch Drücken der Druckknöpfe 56, 66, 76, 86 ist unten mit Bezug auf die Figuren 3 und 4 näher beschrieben.

Die Zugfeder 59 ist so ausgebildet und angeordnet, dass sie die Gewichtskraft der Lenker 46, 47 des bewegbaren Doppelgelenks 51, der horizontalen Armsegmente 60, 70, 80, des distalen Armsegments 90, der Klemmeinrichtung 100 und des Endoskops 16 teilweise, im Wesentlichen vollständig oder vollständig kompensiert. Auch wenn alle Arretierungen der Gelenke 41, 51, 64, 74, 84, 91 gelöst sind, wird die einstellbare Haltevorrichtung 10 deshalb trotzdem nicht oder allenfalls langsam ihre räumliche Gestalt bzw. Konfiguration bzw. Einstellung ändern. Medizinisches Personal, das die einstellbare Haltevorrichtung 10 verstellen will und dazu die Arretierung aller oder fast aller Gelenke löst, muss deshalb nicht im gleichen Moment abrupt die Gewichtskraft der Haltevorrichtung 10 und des Endoskops 16 kompensieren. Durch die vertikale Anordnung der Schwenkachsen 61, 71, 81 hat die Schwerkraft auf diese Freiheitsgrade keine Auswirkung. Aufgrund der Ausgestaltung des Armsegments 40 als Parallelogrammführung gilt dies unabhängig von der mittels des als Parallelogrammführung ausgebildeten Armsegments 40 eingestellten Höhe der Haltevorrichtung 10 bzw. deren distalen Endes 12.

Figur 2 zeigt eine schematische Darstellung einer weiteren einstellbaren Haltevorrichtung 10, die in einigen Merkmalen der oben anhand der Figur 1 dargestellten einstellbaren Haltevorrichtung ähnelt. Einige Merkmale, in denen die in Figur 2 gezeigte einstellbare Haltevorrichtung 10 der oben anhand der Figur 1 dargestellten Haltevorrichtung ähnelt, sind in Figur 2 nicht mit Bezugszeichen versehen und werden nachfolgend auch nicht mehr detailliert beschrieben.

Die in Figur 2 gezeigte einstellbare Haltevorrichtung 10 unterscheidet sich von der oben anhand der Figur 1 dargestellten Haltevorrichtung insbesondere dadurch, dass anstelle eines als Parallelogrammführung ausgebildeten Armsegments zur Höhenverstellung ein mit der Welle 63 starr verbundenes Armsegment 150 vorgesehen ist, das um eine horizontale Schwenkachse 151 eines Gelenkkörpers 152 schwenkbar ist. Der Gelenkkörper 152 ist - ähnlich wie bei dem anhand der Figur 1 dargestellten Beispiel das feststehende Doppelgelenk - in ein Spannfutter 34 einsetzbar, in diesem um eine vertikale Schwenkachse 36 schwenkbar und bezüglich der vertikalen Schwenkachse 36 durch das Spannfutter 34 arretierbar.

Die Schwenkbarkeit des Armsegments 150 um die horizontale Schwenkachse 151 ist arretierbar. Zum selektiven manuellen Lösen der Arretierung des Armsegments 150 sind zwei Druckknöpfe 156 ähnlich den oben in Zusammenhang mit der Ausführungsform der Figur 1 dargestellten Druckknöpfen vorgesehen. Von zwei einander gegenüberliegend angeordneten Druckknöpfen ist in Figur 1 nur einer sichtbar.

Eine Druckfeder 153, beispielsweise eine Gasdruckfeder, stützt das Armsegment 150 gegenüber dem Gelenkkörper 152 ab. Durch die starre Anordnung der ersten vertikalen Welle 63 an dem Armsegment 150 ist eine Höhenverstellung des distalen Endes 12 der einstellbaren Haltevorrichtung 10, und damit beispielsweise eines am distalen Ende 12 der einstellbaren Haltevorrichtung 10 gehaltenen Endoskops 16 in erster Linie durch ein gemeinsames Schwenken des Armsegments 150 und der weiteren Armsegmente 60, 70, 80 um die horizontale Schwenkachse 151 möglich. Eine weitere, begrenzte Höhenverstellung ist - ähnlich wie bei dem oben anhand der Figur 1 dargestellten Beispiel - über das Kugelgelenk 91 möglich.

Die Druckfeder 153 ist ausgebildet und angeordnet, um die Gewichtskraft der einstellbaren Haltevorrichtung - insbesondere die Gewichtskraft der Armsegmente 150, 60, 70, 80, 90, der Klemmeinrichtung 100 und des Endoskops 16 - weitgehend oder vollständig zu kompensieren. Es ist erkennbar, dass durch ein Schwenken in der durch Pfeile 57 angedeuteten im Wesentlichen vertikalen Richtung eine Verstellung der Höhe des distalen Endes 12 der einstellbaren Haltevorrichtung 10 und eines dort gehaltenen Endoskops 16 in einem verhältnismäßig großen Bereich bei einer verhältnismäßig kleinen Abweichung 58 der Schwenkachsen 61, 71, 81 von der Vertikalen möglich ist. Aufgrund der verhältnismäßig kleinen Abweichung 58 der Schwenkachsen 61, 71, 81 von der Vertikalen wirkt die Schwerkraft nur geringfügig auf die entsprechenden Freiheitsgrade.

Insgesamt ermöglicht somit die hier anhand der Figur 2 dargestellte einstellbare Haltevorrichtung auch bei vollständig gelöster Arretierung aller Gelenke verhältnismäßig geringe Haltekräfte, die vom medizinischen Personal aufzubringen sind, um eine Bewegung der einstellbaren Haltevorrichtung zu verhindern. Jedenfalls ist es auch bei der anhand der Figur 2 dargestellten einstellbaren Haltevorrichtung beispielsweise im Gegensatz zu den in der Einleitung erwähnten Haltevorrichtungen mit mehreren Kugelgelenken auch bei vollständig gelöster Arretierung aller Gelenke in der Regel allenfalls erforderlich, einen Bruchteil der Gewichtskraft manuell zu kompensieren.

Bei einer weiteren, zu den Ausführungsformen der Figuren 1 und 2 alternativen, jedoch hier nicht zeichnerisch dargestellten Ausführungsform ist zur Höhenverstellung der einstellbaren Haltevorrichtung eine vertikale Linearführung vorgesehen. Beispielsweise ist ein vertikales Armsegment 30 ähnlich dem vertikalen Armsegment 30 aus Figur 1 oder 2 teleskopierbar ausgebildet. Als Kompensationseinrichtung zum zumindest teilweisen Kompensieren der Schwerkraft der einstellbaren Haltevorrichtung ist beispielsweise eine Gasdruckfeder im Inneren des vertikalen Armsegments vorgesehen.

Bei den Ausführungsformen der Figuren 1 und 2 ist jeweils eine Befestigung an einer Befestigungsschiene 14 vorgesehen. Alternativ kann eine ähnliche einstellbare Haltevorrichtung zur mittelbaren oder unmittelbaren Befestigung an einer Decke eines Operationssaals oder eines anderen Raums ausgebildet sein. Insbesondere kann eine einstellbare Haltevorrichtung zur Befestigung an einer starren oder ihrerseits einstellaren Deckenhalterung ausgebildet sein.

Zur Verwendung bei mittelbarer oder unmittelbarer Befestigung an einer Decke eines Operationssaals können die Ausführungsformen der Figuren 1 und 2 mit wenigen Modifikationen verwendbar sein, beispielsweise indem sie um 180 Grad um eine horizontale Achse gedreht oder an einer horizontalen Ebene gespiegelt werden, also auf den Kopf gestellt werden. Insbesondere ist dazu die Wirkung der Zugfeder 59 der Ausführungsform der Figur 1 bzw. der Druckfeder 153 der Ausführungsform der Figur 2 zu invertieren. Dies kann durch Ersetzen der Zugfeder 59 durch eine Druckfeder bzw. der Druckfeder 153 durch eine Zugfeder oder durch eine Veränderung der Angriffspunkte der Zugfeder 59 bzw. der Druckfeder 153 erfolgen.

Wie bereits erwähnt ist das untere Ende des feststehenden Doppelgelenks insbesondere als Steckbolzen mit einem kreiszylindrischen oder im Wesentlichen kreiszylindrischen Abschnitt ausgebildet. Für eine mittelbaren oder unmittelbaren Befestigung einer einstellbaren Haltevorrichtung an einer Decke eines Operationssaals kann der im Wesentlichen kreiszylindrische Abschnitt eine Kerbe, eine (insbesondere umlaufende) Nut oder einen anderen konkaven Abschnitt aufweisen, um eine formschlüssige Befestigung an einer Deckenhalterung zu ermöglichen.

Die Figuren 3 und 4 zeigen schematische Schnittdarstellungen eines Beispiels einer Gelenkeinrichtung 64, 74, 84 zur Realisierung einer vertikalen Schwenkachse 61, 71, 81 der oben anhand der Figur 1 dargestellten einstellbaren Haltevorrichtung oder der entsprechenden näherungsweise vertikalen Schwenkachsen der einstellbaren Haltevorrichtung aus Figur 2. Die in den Figuren 3 und 4 dargestellten Schnittebenen sind senkrecht zu den Zeichenebenen der Figuren 1 und 2 und senkrecht zu den Schwenkachsen 61, 71, 81.

Auch eine Gelenkeinrichtung an einer der horizontalen Achsen 43, 44, 53, 54 des als Parallelogrammführung ausgebildeten Armsegments 40 der einstellbaren Haltevorrichtung aus Figur 1 und eine Gelenkeinrichtung an der horizontalen Schwenkachse 151 des Armsegments 150 der einstellbaren Haltevorrichtung aus Figur 2 ist insbesondere ähnlich aufgebaut.

Figur 3 zeigt die Gelenkeinrichtung 64, 74, 84 im arretierten Zustand, Figur 4 zeigt die Gelenkeinrichtung 64, 74, 84 bei gelöster Arretierung. Die meisten sich auf gegenständliche Merkmale und Bestandteile der Gelenkeinrichtung 64, 74, 84 beziehenden Bezugszeichen sind nur in Figur 3 enthalten. Figur 4 enthält zusätzliche Bezugszeichen, die sich auf die Funktion der Gelenkeinrichtung 64, 74, 84 beziehen, insbesondere auf Bewegungen von dessen Bestandteilen.

Die Gelenkeinrichtung 64, 74, 84 umfasst ein Gehäuse 111 und eine Antriebseinrichtung 112 in dem Gehäuse 111. Die Antriebseinrichtung 112 umfasst einen kolbenförmigen Abschnitt, der in einem entsprechenden zylindrischen Hohlraum des Gehäuses 121 linear verschiebbar gelagert ist. Zwischen einem äußeren Umfang des kolbenförmigen Abschnitts der Antriebseinrichtung 112 und der gegenüberliegenden Innenwand des Gehäuses 111 ist eine O-Ring-Dichtung 113 vorgesehen. Zwischen einem stabförmigen Abschnitt der Antriebseinrichtung 112 und dem Gehäuse 111 der Gelenkeinrichtung 64, 74, 84 ist eine weitere O-Ring-Dichtung 114 angeordnet. Das Gehäuse 111 und der kolbenförmige Abschnitt der Antriebseinrichtung 112 schließen einen Hohlraum 115 mit variablem Volumen ein, der von den O-Ring-Dichtungen 113, 114 abgedichtet wird.

An der Antriebseinrichtung 112 ist ein Schlauchnippel 116 vorgesehen, über den Druckluft oder ein anderes Fluid in den Hohlraum 115 geleitet oder aus diesem abgeleitet werden kann. Ferner sind Gleitflächen 117 an der Antriebseinrichtung 112 vorgesehen. Eine Druckfeder 118 ist so zwischen der Antriebseinrichtung 112 und dem Gehäuse 111 angeordnet, dass sie die Antriebseinrichtung 112 bezogen auf die Darstellungen in den Figuren 3 und 4 nach links drückt.

An der bezogen auf die Darstellungen in den Figuren 3 und 4 rechten Seite ist das Gehäuse formschlüssig oder auch kraft- oder stoffschlüssig mit einem in gestrichelter Linie angedeuteten Ende eines rohrförmigen Abschnitt 119 eines Armsegments gefügt.

Die Gelenkeinrichtung 64, 74, 84 umfasst ferner eine Arretiereinrichtung 120 mit einer geriffelten Fläche 121 und Gleitflächen 122. Zwischen je einer Gleitfläche 117 an der Antriebseinrichtung 112 und einer Gleitfläche 122 an der Arretiereinrichtung 120 ist jeweils ein Gleitkörper 124 angeordnet. Der Gleitkörper 124 ist insbesondere ein kreiszylindrischer Stab, dessen Zylinderachse senkrecht zur Zeichenebene der Figur ist. Für jeden Gleitkörper ist eine Führung 126 in Form einer weiteren Gleitfläche am Gehäuse 111 vorgesehen. Die Gleitfläche 117 an der Antriebseinrichtung 112 und die Gleitfläche 122 an der Arretiereinrichtung 120, zwischen denen ein Gleitkörper 124 angeordnet ist, sind jeweils nicht parallel, sondern weisen einen zur Führung 126 hin sich erweiternden Abstand auf.

An gegenüberliegenden Seiten der Arretiereinrichtung 120 sind Kugeln 127 angeordnet, die durch Federn 128 gegen nicht parallele Abschnitte der Seitenflächen der Arretiereinrichtung 120 gedrückt werden. Aufgrund der Neigung der erwähnten Abschnitte der Seitenflächen der Arretiereinrichtung 120 üben die Federn 128 über die Kugeln 127 eine Kraft aus, die bezogen auf die Darstellungen in den Figuren 3 und 4 nach rechts wirkt.

Die Gelenkeinrichtung 64, 74, 84 umfasst ferner zwei symmetrisch angelegte Entriegelungseinrichtungen 130. Eine Entriegelungseinrichtung 130 umfasst jeweils eine Kugel 131, die an einer weiteren Gleitfläche 132 an der Antriebseinrichtung 112 anliegen kann. Die Kugel 131 ist jeweils zwischen einem der bereits oben im Zusammenhang mit den Figuren 1 und 2 erwähnten Druckknöpfe 56, 66, 76 und der Gleitfläche 132 an der Antriebseinrichtung 112 angeordnet. Je eine Druckfeder 134 zwischen dem Gehäuse 111 und dem Druckknopf 56, 66, 76 drückt den Druckknopf 56, 66, 76 nach außen.

Figur 3 zeigt die Gelenkeinrichtung 64, 74, 84 in einer Konfiguration bzw. einem Zustand, in dem weder ein mehr als unwesentlicher Druck auf einen der Druckknöpfe 56, 66, 76 ausgeübt wird noch ein gegenüber dem Umgebungsdruck wesentlich erhöhter Druck im Hohlraum 115 zwischen dem kolbenförmigen Abschnitt der Antriebseinrichtung 112 und dem Gehäuse 111 vorliegt. In diesem Zustand drückt die Druckfeder 118 die Antriebseinrichtung 112 bezogen auf die Darstellungen in den Figuren 3 und 4 nach links. Mittels eines Getriebes, das durch die Gleitflächen 117 an der Antriebseinrichtung 112, die Gleitkörper 124 und die Gleitflächen 122 an der Arretiereinrichtung 120 gebildet ist, wird diese Kraft auf die Arretiereinrichtung 120 übertragen, die gegen die Welle 63, 73, 83 gedrückt wird.

Aufgrund der Riffelung der Oberfläche der Welle 63, 73, 83 und der Riffelung der Fläche 121 an der Arretiereinrichtung 120 arretiert die Arretiereinrichtung 120 in dem in Figur 3 gezeigten Zustand die Welle 63, 73, 83 und die Gelenkeinrichtung 64, 74, 84 relativ zueinander. Wenn zwischen der Welle 63, 73, 83 und der Gelenkeinrichtung 64, 74, 84 ein Drehmoment bezogen auf die Schwenkachse 61, 71, 81 ausgeübt wird, wird die Arretiereinrichtung 120 durch die sich in Eingriff befindenden Riffelungen bezogen auf die Darstellungen in den Figuren 3 und 4 nach rechts gedrückt.

Das genannte, durch die Gleitflächen 117 an der Antriebseinrichtung 112, die Gleitkörper 124 und die Gleitflächen 122 an der Arretiereinrichtung 120 gebildete Getriebe ist ausgebildet, um als Sperrgetriebe zu wirken. Die von der Druckfeder 118 zur Aufrechterhaltung der Arretierung aufzubringende Kraft ist deshalb geringer oder deutlich geringer als die Kraft, die die Arretiereinrichtung 120 in der in Figur 3 gezeigten arretierenden Position hält. Dazu sind insbesondere die Gleitflächen 117 an der Antriebseinrichtung 112, die Gleitkörper 124 und die Gleitflächen 122 an der Arretiereinrichtung 120 hinsichtlich Material, Oberflächenbeschaffenheit und relativer Orientierung entsprechend ausgebildet.

Figur 4 zeigt die Gelenkeinrichtung 64, 74, 84 in einem Zustand bzw. einer Konfiguration, bei der die Antriebseinrichtung 112 bezogen auf die Darstellung in Figur 3 nach rechts verschoben ist. Diese Verschiebung nach rechts kann durch einen Zufluss 141 von Druckluft in den Hohlraum 115 und eine resultierende, einen vorbestimmten Mindestbetrag aufweisende oder überschreitende Druckdifferenz zwischen dem Hohlraum 115 und der Umgebung bewirkt werden. Alternativ kann die Verschiebung der Antriebseinrichtung 112 nach rechts durch Druck 142 auf einen oder beide gegenüberliegende Druckknöpfe 56, 66, 76, 86 bewirkt werden. Der Druck 142 gegen die Kraft der jeweiligen Druckfeder 134 verschiebt die Kugel 131 zur Antriebseinrichtung 112. Durch Wechselwirkung der Kugel 131 mit der Gleitfläche 132 an der Antriebseinrichtung 112 wird die Antriebseinrichtung 112 in der durch die Pfeile 143 angedeuteten Richtung nach rechts verschoben.

Durch die Verschiebung der Gleitflächen 117 an der Antriebseinrichtung 112 nach rechts können die Gleitkörper 124 sich aufeinander zu bewegen. Die Gleitkörper 124 setzen den Gleitflächen 122 an der Arretiereinrichtung 120 deshalb keine oder nur noch eine geringe Kraft entgegen. Deshalb können die durch die Federn 128 gegen schräge Abschnitte der Seitenflächen der Arretiereinrichtung 120 gedrückten Kugeln 127 die Arretiereinrichtung 120 aus der arretierenden Position nach rechts verschieben. Dadurch ist die gegenseitige Arretierung von Welle 63, 73, 83 und Gelenkeinrichtung 64, 74, 84 gelöst.

Die Kugeln 127 und die Federn 128 können unter Umständen entfallen, da beispielsweise bei einer Riffelung der Fläche 121 der Arretiereinrichtung 120 und der Oberfläche der Welle 63, 73, 83 mit einem dreieckigen Profil mit schrägen Flanken bereits ein Drehmoment zwischen Welle 63, 73, 83 und Gelenkeinrichtung 64, 74, 84 ausreicht, um die Arretiereinrichtung 120 nach rechts zu verschieben, wenn die Antriebseinrichtung 112 ebenfalls nach rechts verschoben ist.

Ein Getriebe aus einem oder mehreren Gleitkörpern 124, die jeweils zwischen einer Gleitfläche 117 an der Antriebseinrichtung 112 und einer Gleitfläche 122 an der Arretiereinrichtung 120 angeordnet und durch eine Führung 126 geführt sind, ermöglicht eine Übersetzung einer von der Antriebseinrichtung 112 ausgeübten verhältnismäßig kleinen Kraft bei einem verhältnismäßig großem Weg in eine größere Kraft bei kleinerem Weg an der Arretiereinrichtung 120. Wie bereits erwähnt, kann bei Ausnutzung der Reibung zwischen den Gleitkörpern 124 und den Gleitflächen 117, 122 und der Führung 126 das Verhalten eines Sperrgetriebes erzielbar sein, bei dem zum Lösen der Arretierung eine Kraft erforderlich ist, die im Verhältnis zu der von der Druckfeder 118 auf die Antriebseinrichtung 112 ausgewirkten Kraft das einfache, sich im reibungsfreien Fall aus der Geometrie der Gleitkörper 124 und der Gleitflächen 117, 122 und der Führung 126 ergebende Übersetzungsverhältnis bei Weitem übersteigt.

Der Querschnitt des Gehäuses 111 und der Querschnitt der Antriebseinrichtung 112 sind in den Figuren 3 und 4 jeweils einstückig dargestellt. Schon im Sinne eines optimierten Herstellungsaufwands kann es nötig oder vorteilhaft sein, das Gehäuse 111 aus mehreren Stücken zusammenzusetzen. Insbesondere kann es sinnvoll sein, das Gehäuse 111 im Bereich der Führung 126 aus einem harten, abriebfesten Material zu fertigen und ein oder mehrere entsprechende Bauteile in das Gehäuse 111 einzusetzen.

Bei dem anhand der Figuren 3 und 4 dargestellten Beispiel bilden die Druckfeder 118 und die Antriebseinrichtung 112 zusammen einen Antrieb für die Arretierung. Die Arretierung erfolgt durch die Kraft der Druckfeder 118, das Lösen der Arretierung erfolgt pneumatisch oder hydraulisch. Anstelle eines pneumatischen oder hydraulischen Antriebs kann beispielsweise ein elektromotorischer oder linear-magnetischer Antrieb vorgesehen sein. Bei entsprechender Auslegung kann dieser entweder zum Arretieren und zum Lösen der Arretierung oder nur zum Arretieren (gegen die Kraft einer die Arretierung lösenden Feder) oder nur zum Lösen der Arretierung (gegen die Kraft einer arretierenden Feder) vorgesehen sein.

Zum gleichzeitigen Lösen der Arretierungen an allen Gelenkeinrichtungen 64, 74, 84 sind beispielsweise die Schlauchnippel 116 über Schläuche miteinander und mit dem in den Figuren 1 und 2 gezeigten Steuerventil 88 verbunden. Das Steuerventil 88 ist so ausgebildet, dass zum Lösen der Arretierung Druckluft in die Hohlräume 115 geleitet und zum Arretieren aus diesen abgelassen werden kann. Die dazu erforderlichen Schläuche oder Rohre sind in den Figuren 1 und 2 nicht dargestellt und können zumindest teilweise in den Armsegmenten 40, 60, 70, 80, 90 angeordnet sein.

Bei den Ausführungsformen der Figuren 1 und 2 ist die Arretierung des Kugelgelenks 91 am distalen Armsegment 90 lediglich manuell über die Entriegelungshebel 92, die auf das becherförmige Klemmstück 93 wirken, lösbar. Ferner ist bei der Ausführungsform der Figuren 1 und 2 die vertikale Schwenkachse 36, die durch das Spannfutter 34 und das untere, insbesondere als Steckbolzen ausgebildete Ende des feststehenden Doppelgelenks 41 bzw. des Gelenkkörpers 152 definiert ist, lediglich manuell mittels des Spannfutters 34 arretierbar. Diese Arretierung ist auch lediglich durch manuelle Betätigung des Spannfutters 34 lösbar. Beide Ausführungsformen der Figuren 1 und 2 können alternativ jeweils so ausgebildet sein, dass auch das Lösen der Arretierung des Kugelgelenks 91 und/oder das Lösen der Arretierung der vertikalen Schwenkachse 36 mittels des Steuerventils 88 möglich ist.

Insbesondere wenn mittels des Steuerventils 88 auch die Arretierung des Kugelgelenks 91 lösbar ist, kann es vorteilhaft sein, das Steuerventil und einen oder mehrere Druckknöpfe zu seiner Betätigung am distalen Armsegment 90 anzuordnen, beispielsweise anstelle der Entriegelungshebel 92. In diesem Fall kann das Kugelgelenk 91 ähnlich wie oben anhand der Figuren 3 und 4 dargestellt über ein Getriebe betätigt werden, wobei die Arretierung mittels Druckluft gelöst wird. Alternativ sind die Entriegelungshebel 92 ausgebildet, um gleichzeitig unmittelbar auf das becherförmige Klemmstück 93 und auf das Steuerventil zu wirken. In jedem Fall kann es vorteilhaft sein, eine Möglichkeit zum unmittelbaren manuellen Lösen der Arretierung des Kugelgelenks 91 vorzusehen, die ohne Druckluft wirkt.

Wenn die Arretierung der vertikalen Schwenkachse 36, die bei den Ausführungsformen der Figuren 1 und 2 durch das Spannfutter 34 und das untere Ende des feststehenden Doppelgelenks 41 bzw. des Gelenkkörpers 152 definiert ist, mittels des Steuerventils 88 steuerbar sein soll, kann anstelle eines Spannfutters eine Einrichtung ähnlich dem oben anhand der Figuren 3 und 4 dargestellten arretierbaren Gelenks vorgesehen sein. Auch hier kann eine Möglichkeit zum unmittelbaren manuellen Lösen der Arretierung ohne Druckluft vorteilhaft sein.

### Bezugszeichen

- 10: Einstellbare Haltevorrichtung
- 11: proximales Ende der einstellbaren Haltevorrichtung 10
- 12: distales Ende der einstellbaren Haltevorrichtung 10
- 14: Befestigungsschiene an OP-Tisch
- 16: Endoskop (Querschnitt des Schafts)
- 20: Haltesegment
- 22: Klemmeinrichtung am Haltesegment 20
- 24: vertikales Rohr des Haltesegments 20
- 26: Schlitz am vertikalen Rohr 24
- 28: Spanneinrichtung am vertikalen Rohr 24
- 30: vertikales Armsegment
- 32: Druckluftschlauch
- 34: Spannfutter
- 36: vertikale Schwenkachse am vertikalen Armsegment
- 40: als Parallelogrammführung ausgebildetes Armsegment
- 41: feststehendes Doppelgelenk des Armsegments 40
- 43: erste horizontale Schwenkachse am feststehenden Doppelgelenk 41
- 44: zweite horizontale Schwenkachse am feststehenden Doppelgelenk 41
- 46: erster Lenker des Armsegments 40
- 47: zweiter Lenker des Armsegments 40
- 51: verschiebbares Doppelgelenk des Armsegments 40
- 53: dritte horizontale Schwenkachse am verschiebbaren Doppelgelenk 51
- 54: vierte horizontale Schwenkachse am verschiebbaren Doppelgelenk 51
- 56: Druckknopf zur Entriegelung an verschiebbarem Doppelgelenk 51
- 57: Schwenkrichtung der Lenker 46, 47
- 58: Schwenkrichtung des als Parallelogrammführung ausgebildeten Armsegments 40
- 59: Zugfeder zwischen dem ersten Lenker 46 und dem zweiten Lenker 47
- 60: erstes horizontales Armsegment
- 61: erste vertikale Schwenkachse des ersten horizontalen Armsegments
- 63: erste vertikale Welle
- 64: erste Gelenkeinrichtung am ersten horizontalen Armsegment
- 66: Druckknopf zum Lösen einer Arretierung der ersten Gelenkeinrichtung 64
- 70: zweites horizontales Armsegment
- 71: zweite vertikale Schwenkachse des zweiten horizontalen Armsegments
- 73: zweite vertikale Welle
- 74: zweite Gelenkeinrichtung am zweiten horizontalen Armsegment
- 76: Druckknopf zum Lösen einer Arretierung der zweiten Gelenkeinrichtung 74
- 80: drittes horizontales Armsegment
- 81: dritte vertikale Schwenkachse des dritten horizontalen Armsegments
- 83: dritte vertikale Welle am dritten horizontalen Armsegment
- 84: dritte Gelenkeinrichtung am dritten horizontalen Armsegment
- 86: Druckknopf zum Lösen einer Arretierung der dritten Gelenkeinrichtung 84
- 88: Steuerventil
- 90: distales Armsegment
- 91: Kugelgelenk am distalen Armsegment
- 92: Entriegelungshebel am Kugelgelenk
- 93: becherförmiges Klemmstück
- 94: Kupplung
- 100: Klemmeneinrichtung
- 101: feststehende Greifbacke der Klemmeinrichtung 100
- 102: bewegbare Greifbacke der Klemmeinrichtung 100
- 103: Griffhebel an der bewegbaren Greifbacke 102
- 105: Schwenkachse der bewegbaren Greifbacke 102 und des Griffhebels 103

- 111: Gehäuse der Gelenkeinrichtung 64, 74, 84
- 112: Antriebseinrichtung
- 113: O-Ring-Dichtung
- 114: O-Ring-Dichtung
- 115: von der Antriebseinrichtung 112 verschlossener Hohlraum mit variablem Volumen
- 116: Schlauchnippel
- 117: Gleitfläche an der Antriebseinrichtung 112
- 118: Druckfeder zwischen Gehäuse 111 und Antriebseinrichtung 112
- 119: Ende eines rohrförmigen Abschnitt eines Armsegments 60, 70
- 120: Arretiereinrichtung
- 121: geriffelte Fläche an der Arretiereinrichtung 120
- 122: Gleitfläche an der Arretiereinrichtung 120
- 124: Gleitkörper
- 126: Führung für den Gleitkörper 124 am Gehäuse 111
- 127: Kugel
- 128: Feder
- 130: Entriegelungseinrichtung
- 131: Kugel der Entriegelungseinrichtung
- 132: mit der Antriebseinrichtung 112 und der Gleitfläche 117 starr verbundene Gleitfläche
- 134: Druckfeder zwischen Gehäuse 111 und Druckknopf 56, 66, 76, 86
- 141: Zufluss von Druckluft in den Hohlraum 115
- 142: Druck auf den Druckknopf 56, 66, 76, 86
- 143: aus dem Zufluss 141 von Druckluft oder dem Druck 142 auf den Druckknopf 56, 66, 76, 86 resultierende Bewegung der Antriebseinrichtung 112
- 144: aus der Bewegung 143 der Antriebseinrichtung 112 resultierende Bewegung der Arretiereinrichtung 120
- 150: Armsegment
- 151: horizontale Schwenkachse
- 152: Gelenkkörper
- 153: Druckfeder

## Patentansprüche

1. **Einstellbare Haltevorrichtung** (10) für ein Endoskop (16) oder eine andere medizinische Einrichtung, mit:
einem **proximalen Ende** (11);
einem **distalen Ende** (12), mit einer Einrichtung (100) zum Halten eines Endoskops (16) oder einer anderen medizinischen Einrichtung;
einer Mehrzahl von **Armsegmenten** (30, 40, 60, 70, 80) zwischen dem proximalen Ende (11) und dem distalen Ende (12);
einer Mehrzahl von **arretierbaren Gelenken** (51, 64, 74, 84), die jeweils zwei der Mehrzahl von Armsegmenten (30, 40, 60, 70, 80) gelenkig miteinander verbinden;
einer **Kompensationseinrichtung** (59) zum zumindest teilweisen Kompensieren der Gewichtskraft der Haltevorrichtung (10) bei einem manuellen Schwenken oder Verschieben von zumindest einem Armsegment (40) in vertikaler Richtung,
**dadurch gekennzeichnet, dass** ein arretierbares Gelenk (51, 64, 74, 84) eine Arretiereinrichtung (121) zum Arretieren des Gelenks (64, 74, 84), eine Antriebseinrichtung (112, 115, 118) zum Betätigen der Arretiereinrichtung (121) und ein **Getriebe** (117, 122, 124), das eine kleinere Kraft und einen größeren Weg an der Antriebseinrichtung (112, 115, 118) in eine größere Kraft und einen kleineren Weg an der Arretiereinrichtung (121) übersetzt, umfasst,
wobei das Getriebe eine Gleitfläche (122) an der Arretiereinrichtung (121), eine **Gleitfläche** (117) an der Antriebseinrichtung (112, 115, 118), eine **Führung** (126) und ein **Gleitstück** (124), das an der Gleitfläche (122) der Arretiereinrichtung (120) und an der Gleitfläche (117) der Antriebseinrichtung (112, 115, 118) anliegt und durch die Führung (126) geführt ist, umfasst, wobei das Gleitstück (124) eine Kraft von der Gleitfläche (117) auf die Gleitfläche (122) überträgt.

2. Einstellbare Haltevorrichtung (10) nach Anspruch 1, bei der die Kompensationseinrichtung (59) keinen motorischen Antrieb umfasst.

3. Einstellbare Haltevorrichtung (10) nach einem der vorangehenden Ansprüche, bei der das Getriebe (117, 122, 124) ein **Sperrgetriebe** ist.

4. Einstellbare Haltevorrichtung (10) nach einem der vorangehenden Ansprüche, bei der das arretierbare Gelenk (51, 64, 74, 84) ferner eine **Feder** (118) oder ein anderes elastisches Element umfasst, das so mit der Arretiereinrichtung (120) gekoppelt ist, dass eine von dem elastischen Element (118) ausgeübte Kraft eine Arretierung des Gelenks (51, 64, 74, 84) bewirkt.

5. Einstellbare Haltevorrichtung (10) nach einem der vorangehenden Ansprüche, bei der ein Armsegment (40) der Mehrzahl von Armsegmenten (30, 40, 60, 70, 80) in Gestalt einer **Parallelogrammführung** ausgebildet ist.

6. Einstellbare Haltevorrichtung (10) nach dem vorangehenden Anspruch, bei der die Kompensationseinrichtung eine **Feder** (59) oder ein anderes elastisches Element an dem in Gestalt einer Parallelogrammführung ausgebildeten Armsegment (40) umfasst.

7. Einstellbare Haltevorrichtung (10) nach einem der vorangehenden Ansprüche, bei der ein oder mehrere Armsegmente (30, 40, 60, 70, 80) der Mehrzahl von Armsegmenten (30, 40, 60, 70, 80) nur um vertikale oder im Wesentlichen **vertikale Achsen** (61, 71, 81) schwenkbar sind.

8. Einstellbare Haltevorrichtung (10) nach einem der vorangehenden Ansprüche, bei der ein Gelenk (41, 51) an einem vorbestimmten Armsegment (21) ausgebildet ist, ein Schwenken um eine **horizontale Achse** (43, 44) zu ermöglichen, und bei der weitere Gelenke (64, 74, 84) jeweils lediglich für ein Schwenken um eine Achse (61, 71, 81), die **senkrecht zu der horizontalen Achse** (43, 44) ist, ausgebildet sind.

9. Einstellbare Haltevorrichtung (10) nach einem der vorangehenden Ansprüche, ferner mit:
einer **Entriegelungseinrichtung** (130) mit einem Druckknopf (56, 66, 76, 86), die einem einzigen arretierbaren Gelenk (51, 64, 74, 84) zugeordnet ist, zum Lösen der Arretierung des zugeordneten arretierbaren Gelenks (51, 64, 74, 84) durch manuellen Druck auf den Druckknopf (56, 66, 76, 86).

10. Einstellbare Haltevorrichtung (10) nach dem vorangehenden Anspruch, mit einer **Mehrzahl von Entriegelungseinrichtungen** (130), die je einem arretierbaren Gelenk (51, 64, 74, 84) zugeordnet sind.

## Claims

1. Adjustable holder (10) for an endoscope (16) or another medical device, with:
a proximal end (11);
a distal end (12) having a device (100) for holding an endoscope (16) or another medical device;
a plurality of arm segments (30, 40, 60, 70, 80) between the proximal end (11) and the distal end (12);
a plurality of lockable joints (51, 64, 74, 84), each of which connects two of the plurality of arm segments (30, 40, 60, 70, 80) to each other in an articulated manner;
a compensation device (59) for at least partially compensating the weight of the holder (10) during manual pivoting or sliding of at least one arm segment (40) in a vertical direction,
**characterized in that** a lockable joint (51, 64, 74, 84) comprises a locking device (121) for locking the joint (64, 74, 84), a drive device (112, 115, 118) for actuating the locking device (121), and a gear (117, 122, 124) which transmits a smaller force and a greater path on the drive device (112, 115, 118) into a greater force and a smaller path on the locking device (121),
wherein the gear comprises a sliding surface (122) on the locking device (121), a sliding surface (117) on the drive device (112, 115, 118), a guide (126), and a sliding piece (124) which bears on the sliding surface (122) of the locking device (120) and on the sliding surface (117) of the drive device (112, 115, 118) and is guided through the guide (126), wherein the sliding piece (124) transmits a force from the sliding surface (117) to the sliding surface (122).

2. Adjustable holder (10) according to Claim 1, in which the compensation device (59) does not comprise a motor drive.

3. Adjustable holder (10) according to either of the preceding claims, in which the gear (117, 122, 124) is a locking mechanism.

4. Adjustable holder (10) according to one of the preceding claims, in which the lockable joint (51, 64, 74, 84) moreover comprises a spring (118) or another elastic element which is coupled to the locking device (120) in such a way that a force exerted by the elastic element (118) causes a locking of the joint (51, 64, 74, 84).

5. Adjustable holder (10) according to one of the preceding claims, in which an arm segment (40) of the plurality of arm segments (30, 40, 60, 70, 80) is configured in the form of a parallelogram guide.

6. Adjustable holder (10) according to the preceding claim, in which the compensation device comprises a spring (59) or another elastic element on the arm segment (40) configured in the form of a parallelogram guide.

7. Adjustable holder (10) according to one of the preceding claims, in which one or more arm segments (30, 40, 60, 70, 80) of the plurality of arm segments (30, 40, 60, 70, 80) are pivotable only about vertical or substantially vertical axes (61, 71,81).

8. Adjustable holder (10) according to one of the preceding claims, in which a joint (41, 51) is formed on a predetermined arm segment (21) in order to permit pivoting about a horizontal axis (43, 44), and in which further joints (64, 74, 84) are each configured for pivoting only about an axis (61, 71, 81) that is perpendicular to the horizontal axis (43, 44).

9. Adjustable holder (10) according to one of the preceding claims, moreover with:
an unlocking device (130) with a pushbutton (56, 66, 76, 86), which unlocking device (130) is assigned to a single lockable joint (51, 64, 74, 84) in order to release the locking of the assigned lockable joint (51, 64 74, 84) by manual pressure on the pushbutton (56, 66, 76, 86).

10. Adjustable holder (10) according to the preceding claim, with a plurality of unlocking devices (130), each assigned to a lockable joint (51, 64, 74, 84).

## Revendications

1. Arrangement de maintien réglable (10) pour un endoscope (16) ou un autre dispositif médical, comprenant :
une extrémité proximale (11) ;
une extrémité distale (12), munie d'un dispositif (100) servant à maintenir un endoscope (16) ou un autre dispositif médical ;
une pluralité de segments de bras (30, 40, 60, 70, 80) entre l'extrémité proximale (11) et l'extrémité distale (12) ;
une pluralité d'articulations blocables (51, 64, 74, 84) qui relient ensemble de manière articulée respectivement deux de la pluralité de segments de bras (30, 40, 60, 70, 80) ;
un dispositif de compensation (59) destiné à compenser au moins partiellement le poids de l'arrangement de maintien (10) lors d'un pivotement ou d'un déplacement manuel d'au moins un segment de bras (40) dans la direction verticale,
**caractérisé en ce qu'**une articulation blocable (51, 64, 74, 84) comporte un dispositif de blocage (121) destiné à bloquer l'articulation (64, 74, 84), un dispositif d'entraînement (112, 115, 118) destiné à actionner le dispositif de blocage (121) et un engrenage (117, 122, 124) qui convertit une force plus petite et une course plus grande au niveau du dispositif d'entraînement (112, 115, 118) en une force plus grande et une course plus petite au niveau du dispositif de blocage (121),
l'engrenage comportant une surface de glissement (122) au niveau du dispositif de blocage (121), une surface de glissement (117) au niveau du dispositif d'entraînement (112, 115, 118), un guide (126) et une pièce coulissante (124) qui repose sur la surface de glissement (122) du dispositif de blocage (121) et sur la surface de glissement (117) du dispositif d'entraînement (112, 115, 118) et qui est guidée par le guide (126), la pièce coulissante (124) transmettant une force de la surface de glissement (117) à la surface de glissement (122).

2. Arrangement de maintien réglable (10) selon la revendication 1, le dispositif de compensation (59) ne comportant pas de mécanisme d'entraînement motorisé.

3. Arrangement de maintien réglable (10) selon l'une des revendications précédentes, l'engrenage (117, 122, 124) étant un engrenage à verrouillage.

4. Arrangement de maintien réglable (10) selon l'une des revendications précédentes, l'articulation blocable (51, 64, 74, 84) comportant en outre un ressort (118) ou un autre élément élastique qui est accouplé au dispositif de blocage (120) de telle sorte qu'une force exercée par l'élément élastique (118) provoque un blocage de l'articulation (51, 64, 74, 84).

5. Arrangement de maintien réglable (10) selon l'une des revendications précédentes, avec lequel un segment de bras (40) de la pluralité de segments de bras (30, 40, 60, 70, 80) est réalisé sous la forme d'un guide à parallélogramme.

6. Arrangement de maintien réglable (10) selon la revendication précédente, avec lequel le dispositif de compensation comporte un ressort (59) ou un autre élément élastique au niveau du segment de bras (40) réalisé sous la forme d'un guide à parallélogramme.

7. Arrangement de maintien réglable (10) selon l'une des revendications précédentes, avec lequel un ou plusieurs segments de bras (30, 40, 60, 70, 80) de la pluralité de segments de bras (30, 40, 60, 70, 80) peuvent seulement pivoter autour d'axes verticaux (61, 71, 81) ou sensiblement verticaux.

8. Arrangement de maintien réglable (10) selon l'une des revendications précédentes, avec lequel une articulation (41, 51) au niveau d'un segment de bras (21) prédéfini est configurée pour permettre un pivotement autour d'un axe horizontal (43, 44), et avec lequel des articulations supplémentaires (64, 74, 84) sont respectivement configurées uniquement pour un pivotement autour d'un axe (61, 71, 81) qui est perpendiculaire à l'axe horizontal (43, 44).

9. Arrangement de maintien réglable (10) selon l'une des revendications précédentes, comprenant en outre :
un dispositif de déverrouillage (130) muni d'un bouton-poussoir (56, 66, 76, 86) qui est associé à une seule articulation blocable (51, 64, 74, 84) pour libérer le blocage de l'articulation blocable (51, 64, 74, 84) associée par une pression manuelle sur le bouton-poussoir (56, 66, 76, 86).

10. Arrangement de maintien réglable (10) selon l'une des revendications précédentes, comprenant une pluralité de dispositifs de déverrouillage (130) qui sont respectivement associés à une articulation blocable (51, 64, 74, 84).
